(19) Europäisches Patentamt
European Patent Office
Office européen des brevets

(11) Veröffentlichungsnummer: **0 304 018**
**A2**

(12) **EUROPÄISCHE PATENTANMELDUNG**

(21) Anmeldenummer: **88113309.4**

(51) Int. Cl.4: **C07D 209/48**

(22) Anmeldetag: **17.08.88**

(30) Priorität: **21.08.87 DE 3727898**

(43) Veröffentlichungstag der Anmeldung:
**22.02.89 Patentblatt 89/08**

(84) Benannte Vertragsstaaten:
**AT BE CH DE ES FR GB IT LI NL SE**

(71) Anmelder: **Degussa Aktiengesellschaft**
**Weissfrauenstrasse 9**
**D-6000 Frankfurt am Main 1(DE)**

(72) Erfinder: **Effenberger, Franz, Prof.**
**Schatzweg 5**
**D-7000 Stuttgart 70(DE)**
Erfinder: **Steegmüller, Dieter, Dr.**
**Warmbronner Strasse 13/1**
**D-7031 Magstadt(DE)**

(54) **Verfahren zur Herstellung von Aryl-(1-phthalimido)-alkylketonen.**

(57) Aryl-(1-phthalimido)-alkylketone der allgemeinen Formel

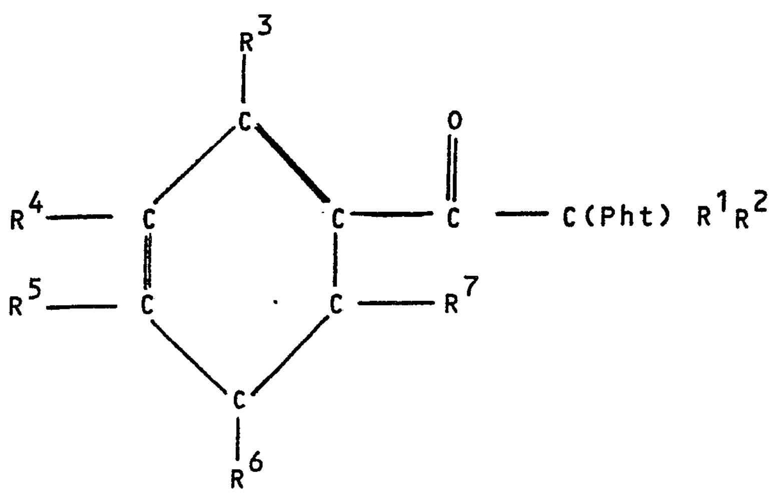

werden dadurch hergestellt, daß man eine geeignete aromatische Verbindung mit mindestens einem Wasserstoffatom am aromatischen Kern in einem inerten Lösungsmittel und in Gegenwart von Eisen-(III)-chlorid mit einem N-Phthaloyl-2-aminocarbonsäurechlorid der allgemeinen Formel

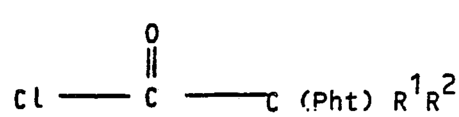

umsetzt. Sie können durch Reduktion der Ketogruppe in entsprechende 2-Phenethanolamine oder 2-

EP 0 304 018 A2

Phenethylamine umgewandelt werden.

## Verfahren zur Herstellung von Aryl-(1-phthalimido)alkylketonen

Die Erfindung betrifft ein Verfahren zur Herstellung von Aryl-(1-phthalimido)-alkylketonen der allgemeinen Formel

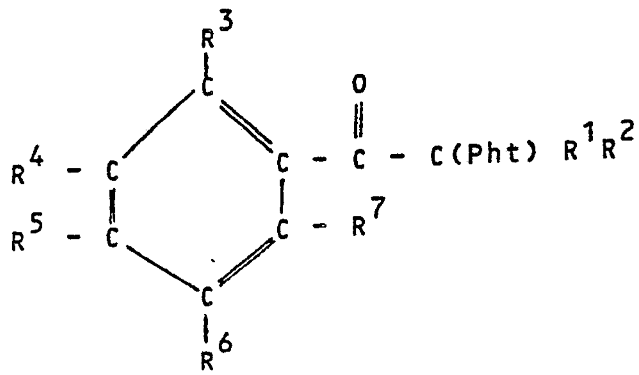

(I),

in der
$R^1$ Wasserstoff oder einen geradkettigen oder verzweigten ($C_1$-$C_4$)-Alkylrest,
$R^2$ Wasserstoff, einen geradkettigen oder verzweigten ($C_1$-$C_6$)-Alkylrest, einen Benzylrest, einen 4-($C_1$-$C_4$)-Alkoxybenzylrest, einen 4-Benzyloxylbenzylrest, einen 3,4-Di-($C_1$-$C_4$)-alkoxybenzylrest, einen 1,3-Benzodioxol-5-yl-methylrest, einen N-Formyl-indol-3-yl-methylrest, einen N-($C_2$-$C_3$)-Acylindol-3-yl-methylrest, einen 5-($C_1$-$C_4$)-Alkoxy-N-($C_2$-$C_3$)-acyl-indol-3-yl-methylrest, einen 5-($C_1$-$C_4$)-Alkoxy-N-formyl-indol-3-yl-methylrest oder einen durch eine ($C_1$-$C_6$)-Alkoxygruppe, eine Benzyloxygruppe, eine Phenyloxygruppe, eine Phthalimidogruppe, eine ($C_1$-$C_4$)- Alkoxycarbonylgruppe oder eine Benzyloxy-carbonylgruppe substituierten ($C_1$-$C_4$)-Alkylrest.
$R^3$,$R^4$,$R^5$,$R^6$ und $R^7$ unabhängig voneinander Wasserstoff, einen geradkettigen oder verzweigten ($C_1$-$C_4$)-Alkylrest, einen ($C_1$-$C_6$)-Alkoxyrest, einen Benzyloxyrest oder einen Phenyloxyrest und
Pht einen Phthalimidorest bedeuten.

Aryl-(1-phthalimido)-alkylketone sind Ausgangsstoffe für die Herstellung pharmakologisch interessanter Wirkstoffe, die sich vom 2-Phenylethylamin ableiten. So können durch Reduktion der Ketogruppe chirale 2-Phenethanolamine oder chirale 2-Phenethylamine hergestellt werden [JACS 103, 6157 (1981)].

Die Herstellung von N-geschützten Aryl-(1-amino)-alkylketonen erfolgt üblicherweise durch Friedel-Crafts-Acylierung aromatischer Verbindungen mit geeigneten N-geschützten 2-Aminocarbonsäure-Derivaten. Versuche, Alkoxy-substituierte aromatische Verbindungen mit N-geschützten 2-Aminocarbonsäurechloriden nach Friedel-Crafts zu acylieren, versagen jedoch im allgemeinen infolge der Komplexierung des Aluminiumchlorids mit den Alkoxy-Substituenten und der dadurch bedingten Desaktivierung der aromatischen Verbindungen [JACS 103, 6157 (1981); J.Org. Chem. 48, 2675 (1983)].

Das erfindungsgemäße Verfahren ist nun dadurch gekennzeichnet, daß man eine aromatische Verbindung der allgemeinen Formel

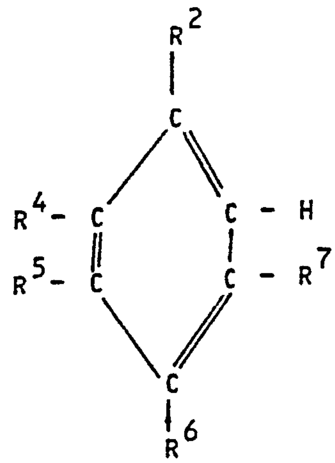

(II),

in der $R^3$, $R^4$, $R^5$, $R^6$ und $R^7$ jeweils eine der bereits genannten Bedeutungen aufweisen, in einem inerten Lösungsmittel und in Gegenwart von Eisen-(III)-chlorid mit einem N-Phthaloyl-2-aminocarbonsäurechlorid der allgemeinen Formel

$$Cl - \overset{O}{\overset{\|}{C}} - C\,(Pht)\,R^1R^2 \qquad (III),$$

in der $R^1$,$R^2$ und Pht die bereits genannten Bedeutungen aufweisen, umsetzt.

Erstaunlicherweise gelingt mit dem erfindungsgemäßen Verfahren gerade die Umsetzung von Alkoxy-substituierten aromatischen Verbindungen besonders gut. Während bei üblichen Friedel-Crafts-Acylierungen die 1- bis 3-fache molare Menge an Aluminiumchlorid, bezogen auf das eingesetzte Acylierungsmittel, angewandt werden muß, genügen beim erfindungsgemäßen. Verfahren schon sehr viel geringere katalytische Mengen an Eisen-(III)-chlorid. Vorzugsweise wird das Eisen-(III)-chlorid in einer Menge zwischen 0,5 und 50 Molprozent, bezogen auf das eingesetzte N-Phthaloyl-2-aminocarbonsäurechlorid der allgemeinen Formel (III) eingesetzt.

Die Umsetzung der aromatischen Verbindung der allgemeinen Formel (II) mit dem N-Phthaloyl-2-aminocarbonsäurechlorid der allgemeinen Formel (III) wird zweckmäßigerweise bei einer Temperatur im Bereich von -10 bis 80 °C vorgenommen.

Geeignete inerte Lösungsmittel für die Durchführung des erfindungsgemäßen Verfahrens sind beispielsweise chlorierte aliphatische Kohlenwasserstoffe, wie Methylenchlorid, Chloroform, Tetrachlorkohlenstoff, 1,2-Dichlorethan oder Trichlorethylen, Alkane, wie Hexan , Heptan oder Cyclohexan; und Schwefelkohlenstoff.

Eine besonders zweckmäßige Ausführungsform des erfindungsgemäßen Verfahrens besteht darin, daß man das N-Phthaloyl-2-aminocarbonsäurechlorid der allgemeinen Formel (III) in dem inerten Lösungsmittel aus der entsprechenden N-Phthaloyl-2-aminocarbonsäure herstellt und dann das Eisen-(III)-chlorid und die aromatische Verbindung der allgemeinen Formel (II) hinzugibt.

Das N-Phthaloyl-2-aminocarbonsäurechlorid der allgemeinen Formel (III) kann beispielsweise so hergestellt werden, daß man die entsprechende N-Phthaloyl-2-aminocarbonsäure in dem inerten Lösungsmittel auflöst und sie mit einem dafür geeigneten Reagenz, z.B. Phosphorpentachlorid, in das Säurechlorid umwandelt. Zu der so erhaltenen Lösung des N-Phthaloyl-2-aminocarbonsäurechlorids der allgemeinen Formel (III) wird dann das Eisen-(III)-chlorid und die aromatische Verbindung der allgemeinen Formel (II) hinzugegeben und es wird bis zur vollständigen Umsetzung des Säurechlorids gerührt. Alternativ hierzu kann auch die Lösung des N-Phthaloyl-2-aminocarbonsäurechlorids der allgemeinen Formel (III) zu einer vorgelegten Lösung des Eisen-(III)-chlorids und der aromatischen Verbindung der allgemeinen Formel (II) zudosiert werden.

Zur Aufarbeitung wird das Reaktionsgemisch zweckmäßigerweise auf 0 °C abgekühlt und mit gesättigter wäßriger Natriumhydrogencarbonat-Lösung oder wäßriger Salzsäure versetzt. Die sich dabei bildenden zwei Phasen werden getrennt, und die organische Phase wird mit Wasser ausgewaschen. Das gebildete Aryl-(1-phthalimido)-alkylketon der allgemeinen Formel (I) wird durch Kristallisation aus der organischen Phase oder durch an sich bekannte säulenchromatographische Methoden rein erhalten.

Bei der Umsetzung von chiralen N-Phthaloyl-2-aminocarbonsäurechloriden der allgemeinen Formel (III) weist das erfindungsgemäße Verfahren den Vorteil auf, daß die Umsetzung weitgehend unter Erhalt des Chiralitätszentrums verläuft.

Die Erfindung wird durch die nachfolgenden Beispiele näher erläutert. Die Enantiomerenreinheit der erhaltenen Produkte wurde $^{1}$H-NMR-spektroskopisch unter Verwendung des Shiftreagenz Eu(hfbc)3 nachgewiesen.

Beispiel 1:

Eine Lösung von 2,19 g (10,0 mMol) N-Phthaloyl-(S)-alanin in 30 ml 1,2-Dichlorethan wurde bei 0 °C mit 2,29 g (11,0 mMol) Phosphorpentachlorid versetzt und bei Raumtemperatur 4 Stunden lang gerührt. Nach Zugabe von 20 ml Mesitylen und 16 mg Eisen-(III)-chlorid (1 Molprozent) wurde bei 25 °C 48 Stunden lang gerührt, dann auf 0 °C abgekühlt und mit 50 ml gesättigter wäßriger Natriumhydrogencarbonat-Lösung und 20 ml Aceton versetzt. Die wäßrige Phase wurde abgetrennt und die organische Phase wurde zweimal mit jeweils 40 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck kristallisierte das Produkt beim Stehenlassen im Kühlschrank. Es wurde über Paraffinschnitzeln getrocknet. Es wurden 2,54 g (79 % der Theorie) analysenreines (S)-2,4,6-Trimethylphenyl-(1-phthalimidoethyl)keton mit einem Schmelzpunkt von 142-143 °C erhalten.

$[\alpha]_D^{20}$ : +52,8° (c = 2, $CHCl_3$)

Beispiel 2:

Eine Lösung von 2,61 g (10,0 mMol) N-Phthaloylleucin in 30 ml 1,2-Dichlorethan wurde bei 0 °C mit 2,29 g (11,0 mMol) Phosphorpentachlorid versetzt und bei Raumtemperatur 4 Stunden lang gerührt. Nach Zugabe von 20 ml Mesitylen und 16 mg Eisen-(III)-chlorid (1 Molprozent) wurde bei 50 °C 40 Stunden lang gerührt, dann auf 0 °C abgekühlt und mit 50 ml gesättigter wäßriger Natriumhydrogencarbonat-Lösung und 20 ml Aceton versetzt und 4 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 40 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck kristallisierte das Produkt beim Stehenlassen im Kühlschrank. Es wurde über Paraffinschnitzeln getrocknet. Es wurden 2,14 g (56 % der Theorie) analysenreines 2,4,6-Trimethylphenyl-(3-methyl-1-phthalimidobutyl)-keton mit einem Schmelzpunkt von 131-132 °C erhalten.

Beispiel 3:

Eine Lösung von 2,19 g (10,0 mMol) N-Phthaloylalanin in 30 ml 1,2-Dichlorethan wurde bei 0 °C mit 2,29 g (11,0 mMol) Phosphorpentachlorid versetzt und bei Raumtemperatur 4 Stunden lang gerührt. Nach Zugabe von 20 ml p-Xylol und 16 mg Eisen-(III)-chlorid (1 Molprozent) wurde bei 25 °C 48 Stunden lang gerührt, dann auf 0 °C abgekühlt und mit 50 ml gesättigter wäßriger Natriumhydrogencarbonat-Lösung und 20 ml Aceton versetzt und 4 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 40 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck kristallisierte das Produkt beim Stehenlassen im Kühlschrank. Es wurde über Paraffinschnitzeln getrocknet. Es wurden 1,29 g (42 % der Theorie) analysenreines 2,4-Dimethylphenyl-(1-phthalimidoethyl)-keton mit einem Schmelzpunkt von 112 °C erhalten.

Beispiel 4:

Eine Lösung von 4,38 g (20,0 mMol) N-Phthaloyl-(S)-alanin in 120 ml 1,2-Dichlorethan wurde bei 0 °C mit 4,58 g (22,0 mMol) Phosphorpentachlorid versetzt und bei Raumtemperatur 4 Stunden lang gerührt. Nach Zusatz von weiteren 120 ml 1,2-Dichlorethan wurde ein Zehntel der Lösung schnell zu einer gerührten Lösung von 3,32 g (20,0 mMol) Hydrochinondiethylether und 320 mg Eisen- (III)-chlorid (10 Molprozent) in 80 ml 1,2 Dichlorethan zugegeben. Das Reaktionsgemisch wurde auf 50 °C erwärmt, und die restliche

Lösung des Säurechlorids wurde kontinuierlich innerhalb von 12 Stunden zugetropft. Anschließend wurde weitere 12 Stunden bei 50 °C gerührt, dann auf 0 °C abgekühlt. Es wurden 100 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 150 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Produkt über eine Kieselgelsäule chromatographiert. Nach erneutem Entfernen des Lösungsmittels kristallisierte das Produkt beim Stehenlassen im Kühlschrank. Es wurden 5,33 g (73 % der Theorie) (S)-2,5-Diethoxyphenyl-(1-phthalimidoethyl)-keton mit einem Schmelzpunkt von 92-93 °C erhalten.

$[\alpha]_D^{20}$ : + 25,9° (c = 2, $CHCl_3$)

Beispiel 5:

Eine Lösung von 2,61 g (10,0 mMol) N-Phthaloylleucin in 60 ml 1,2-Dichlorethan wurde bei 0 °C mit 2,29 g (11,0 mMol) Phosphorpentachlorid versetzt und zunächst 30 Minuten lang bei 0 °C, dann 4 Stunden lang bei Raumtemperatur gerührt. Nach Zusatz von weiteren 60 ml 1,2-Dichlorethan wurde ein Zehntel der Lösung schnell zu einer gerührten Lösung von 1,66 g (10,0 mMol) Hydrochinondiethylether und 160 mg Eisen-(III)-chlorid (10 Molprozent) in 40 ml 1,2-Dichlorethan zugegeben. Das Reaktionsgemisch wurde auf 50 °C erwärmt, und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 100 ml auf 0 °C abgekühlte 1N Salzsäure zugesetzt und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Produkt über eine Kieselgelsäule chromatographiert. Es wurden 2,53 g (62 % der Theorie) analysenreines 2,5-Diethoxyphenyl-(3-methyl-1-phthalimidobutyl)-keton als Öl erhalten.

Beispiel 6:

Eine Lösung von 4,38 g (20,0 mMol) N-Phthaloyl-(S)-alanin in 120 ml 1,2-Dichlorethan wurde bei 0 °C mit 4,58 g (22,0 mMol) Phosphorpentachlorid versetzt und zunächst 30 Minuten lang bei 0 °C, dann 4 Stunden lang bei Raumtemperatur gerührt. Nach Zusatz von weiteren 120 ml 1,2-Dichlorethan wurde ein Zehntel der Lösung schnell zu einer gerührten Lösung von 2,76 g (20,0 mMol) Brenzcatechindimethylether und 320 mg Eisen-(III)-chlorid (10 Molprozent) in 80 ml 1,2-Dichlorethan zugegeben. Das Reaktionsgemisch wurde auf 50 °C erwärmt , und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 100 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 150 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Produkt aus Ethanol umkristallisiert Es wurden 3,71 g (55 % der Theorie) analysenreines (S)-3,4-Dimethyoxyphenyl-(1-phthalimidoethyl)-keton mit einem Schmelzpunkt von 126-128 °C erhalten.

$[\alpha]_D^{20}$ : -152,5° (c = 1,2, $CHCl_3$)

Beispiel 7:

Eine Lösung von 2,61 g (10,0 mMol) N-Phthaloyl-(S)-leucin in 60 ml 1,2-Dichlorethan wurde bei 0 °C mit 2,29 g (11,0 mMol) Phosphorpentachlorid versetzt und zunächst 30 Minuten lang bei 0 °C, dann 4 Stunden lang bei Raumtemperatur gerührt. Nach Zusatz von weiteren 60 ml 1,2-Dichlorethan wurde ein Zehntel der Lösung schnell zu einer gerührten Lösung von 1,38 g (10,0 mMol) Brenzcatechindimethylether und 160 mg Eisen-(III)-chlorid (10 Molprozent) in 40 ml 1,2-Dichlorethan zugegeben. Das Reaktionsgemisch

wurde auf 50 °C erwärmt, und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 50 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Produkt über eine Kieselgelsäule chromatographiert. Es wurden 2,11 g (55 % der Theorie) analysenreines (S)-3,4-Dimethoxyphenyl-(3-methyl-1-phthalimidobutyl)-keton mit einem Schmelzpunkt von 128,5 °C erhalten.

$[\alpha]_D^{20}$ : -67,2 ° (c = 1, $CHCl_3$)

Beispiel 8:

Eine Lösung von 4,38 g (20,0 mMol) N-Phthaloyl-(S)-alanin in 60 ml 1,2-Dichlorethan wurde bei 0 °C mit 4,58 g (22,0 mMol) Phosphorpentachlorid versetzt und zunächst 30 Minuten lang bei 0 °C, dann 4 Stunden lang bei Raumtemperatur gerührt. Nach Zusatz von weiteren 60 ml 1,2-Dichlorethan wurde ein Zehntel der Lösung schnell zu einer gerührten Lösung von 2,16 g (20,0 mMol) Anisol und 320 mg Eisen-(III)-chlroid (10 Molprozent) in 80 ml 1,2-Dichlorethan zugegeben. Das Reaktionsgemisch wurde auf 50 °C erwärmt, und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 100 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 150 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Rohprodukt über eine Kieselgelsäule chromatographiert. Das erhaltene ölige Isomerengemisch (4,56 g = 74 % der Theorie) wurde in heißem Toluol gelöst, und die Lösung wurde bis zur beginnenden Trübung mit n-Hexan versetzt. Dabei fielen 2,14 g (35 % der Theorie) analysenreines (S)-4-Methoxyphenyl-(1-phthalimidoethyl)-keton mit einem Schmelzpunkt von 110 °C aus.

$[\alpha]_D^{20}$ : -109,1 ° (c = 2, $CHCl_3$)

Beispiel 9:

Eine Lösung von 2,19 g (10,0 mMol) n Phthaloyl-(S)-alanin in 30 ml 1,2-Dichlorethan wurde bei 0 °C mit 2,29 g (11,0 mMol) Phosphorpentachlorid versetzt und zunächst 30 Minuten lang bei 0 °C, dann 4 Stunden lang bei Raumtemperatur gerührt. Nach Zusatz von weiteren 30 ml 1,2-Dichlorethan wurde ein Zehntel der Lösung schnell zu einer gerührten Lösung von 1,22 g (10,0 mMol) Ethoxybenzol und 160 mg Eisen-(III)-chlorid (10 Molprozent) in 40 ml 1,2-Dichlorethan zugegeben. Das Reaktionsgemisch wurde auf 50 °C erwärmt, und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 50 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit Wasser gewaschen und über Natriumsulfat getrocknet. Das Rohprodukt wurde über eine Kieselgelsäule chromatographiert. Von dem erhaltenen öligen Isomerengemisch (1,82 g = 56 % der Theorie) wurden 200 mg über MPLC aufgetrennt. Dabei fielen 50 mg (14 % der Theorie) analysenreines (S)-2-Ethoxyphenyl-(1-phthalimidoethyl)-keton als farbloses Öl {$[\alpha]_D^{20}$ : +59,6 % (c = 1, $CHCl_3$)} und 96 mg (26 % der Theorie) analysenreines (S)-4-Ethoxyphenyl-(1-phthalimidoethyl)-keton als farbloses Öl an {$[\alpha]_D^{20}$ : -74 °C (c = 2, $CHCl_3$)}.

Beispiel 10:

Eine Lösung von 2,19 g (10,0 mMol) N-Phthaloyl-(S)-alanin in 30 ml 1,2-Dichlorethan wurde bei 0 °C mit 2,29 g (11,0 mMol) Phosphorpentachlorid versetzt und zunächst 30 Minuten lang bei 0 °C, dann 4 Stunden lang bei Raumtemperatur gerührt. Nach Zusatz von weiteren 30 ml 1,2-Dichlorethan wurde ein Zehntel der Lösung schnell zu einer gerührten Lösung von 1,50 g (10,0 mMol) n-Butoxylbenzol und 160 mg Eisen-(III)-chlorid (10 Molprozent) in 40 ml 1,2-Dichlorethan zugegeben. Das Reaktionsgemisch wurde auf 50 °C erwärmt, und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 50 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit Wasser gewaschen und über Natriumsulfat getrocknet. Das Rohprodukt wurde über eine Kieselgelsäule chromatographiert. Von dem erhaltenten öligen Isomerengemisch (2,01 g = 57 % der Theorie) wurden 170 mg über MPLC aufgetrennt. Dabei fielen 40 mg (13 % der Theorie) analysenreines (S)-2-Butoxyphenyl-(1-phthalimidoethyl)-keton als farbloses Öl {$[\alpha]_D^{20}$ : + 36,3° (c= 0,8 $CHCl_3$)} und 64 mg (22 % der Theorie) analysenreines (S)-4-Butoxyphenyl-(1-phthalimidoethyl)-keton als farbloses Öl an {$[\alpha]_D^{20}$ : -63,2° (c = 1,2, $CHCl_3$)}

Beispiel 11:

Eine Lösung von 2,47 g (10,0 mMol) N-Phthaloyl-(S)-valin in 60 ml 1,2-Dichlorethan wurde bei 0 °C mit 2,29 g (11,0) mMol) Phosphorpentachlorid versetzt und zunächst 30 Minuten lang bei 0 °C, dann 4 Stunden lang bei Raumtemperatur gerührt. Nach Zusatz von weiteren 60 ml 1,2-Dichlorethan wurde ein Zehntel der Lösung schnell zu einer gerührten Lösung von 1,38 g (10,0 mMol) Brenzcatechindimethylether und 160 mg Eisen-(III)-chlorid (10 Molprozent) in 40 ml 1,2-Dichlorethan zugegeben. Das Reaktionsgemisch wurde auf 50 °C erwärmt, und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 50 ml auf 0 °C aufgekühlte 1N Salzsäure zugesetzt, und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Produkt über eine Kieselgelsäule chromatographiert. Es wurden 2,16 g (59 % der Theorie) analysenreines (S)-3,4-Dimethoxyphenyl-(2-methyl-1-phthalimidopropyl)-keton als farbloses Öl erhalten.

$[\alpha]_D^{20}$ : -200,4° (c = 1, $CHCl_3$).

Beispiel 12:

Eine Lösung von 4,06 g (10,0 mMol ) N,N′-Diphthaloyl-(S)-lysin in 60 ml 1,2 Dichlorethan wurde bei 0 °C mit 2,29 g (11,0 mMol) Phosphorpentachlorid versetzt und zunächst 30 Minuten lang bei 0 °C, dann 4 Stunden lang bei Raumtemperatur gerührt. Nach Zusatz von weiteren 60 ml 1,2-Dichlorethan wurde ein Zehntel der Lösung schnell zu einer gerührten Lösung von 1,38 g (10,0 mMol) Brenzcatechindimethylether und 160 mg Eisen-(III)-chlorid (10 Molprozent) in 40 ml 1,2-Dichlorethan zugegeben. Das Reaktionsgemisch wurde auf 50 °C erwärmt und die restliche Lösung des Säurechlorids wurde kontinuierlich innerhalb von 12 Stunden zugetropft. Anschließend wurde weitere 12 Stunden lang bei 50 °C gerührt, dann auf 0 °C abgekühlt. Es wurden 50 ml auf 0 °C abgekühlte 1N Salzsäure zugesetzt, und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit Wasser gewaschen und über Natriumsulfat getrocknet. Nach Entfernen der Lösungsmittel bei 40 °C unter vermindertem Druck wurde das Produkt über eine Kieselgelsäule chromatographiert. Es wurden 3,26 g (62 % der Theorie) analysenreines (S)-3,4-Dimethoxyphenyl-(1,5-bisphthalimidopentyl)-keton mit einem Schmelzpunkt von 195 °C erhalten.

$[\alpha]_D^{20}$ : -81,1° (c = 1,1, $CHCl_3$).

Beispiel 13:

Eine Lösung von 2,95 g (10,0 mMol) N-Phthaloyl-(S)-phenylalanin in 50 ml 1,2-Dichlorethan wurde bei 0° C mit 2,29 g (11,0 mMol) Phosphorpentachlorid versetzt und zunächst 30 Minuten lang bei 0° C, dann 16 Stunden lang bei Raumptemperatur gerührt. Nach Zusatz von weiteren 50 ml 1,2-Dichlorethan wurde ein Zehntel der Lösung schnell zu einer gerührten Lösung von 1,38 g (10,0 mMol) Brenzcatechindimethylether und 160 mg Eisen-(III)-chlorid (10 Molprozent) in 70 ml 1,2-Dichlorethan zugegeben. Das Reaktionsgemisch wurde auf 50° C erwärmt, und die restliche Lösung des Säurechlorids wurde kontinuierlich innerhalb von 12 Stunden zugetropft. Anschließend wurde weitere 12 Stunden lang bei 50° C gerührt, dann auf 0° C abgekühlt. Es wurden 100 ml auf 0° C abgekühlte 1N Salzsäure zugesetzt, und es wurde 24 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 150 ml gesättigter wäßriger Natriumhydrogencarbonat-Lösung, dann mit 100 ml Wasser gewaschen und über Natriumsulfat getrocknet. Nach Entfernen der Lösungs mittel bei 40° C unter vermindertem Druck wurde das Rohprodukt über eine Kieselgelsäule chromatographiert. Es wurden 2,45 g (59 % der Theorie) analysenreines (S)-3,4-Dimethoxyphenyl-(2-phenyl-1-phthalimidoethyl)-keton als Öl erhalten.

$[\alpha]_D^{20}$ : -245° (c = 1, $CHCl_3$).

Als Nebenprodukt konnten 0,25 g (9 % der Theorie) analysenreines 2-Phthalimido-1-indanon mit einem Schmelzpunkt von 198 - 199° C isoliert werden.

Beispiel 14:

Eine Lösung von 1,55 g (5,0 mMol) N-Phthaloyl-2-methylphenylalanin in 25 ml 1,2-Dichlorethan wurde bei 0° C mit 1,15 g (5,50 mMol) Phosphorpentachlorid versetzt und zunächst 30 Minuten lang bei 0° C, dann 16 Stunden lang bei Raumtemperatur gerührt. Nach Zusatz von weiteren 25 ml 1,2-Dichlorethan wurde ein Zehntel der Lösung schnell zu einer gerührten Lösung von 0,69 g (5,0 mMol) Brenzcatechindimethylether und 80 mg Eisen-(III)-chlorid (10 Molprozent) in 40 ml 1,2-Dichlorethan zugegeben. Das Reaktionsgemisch wurde auf 50° C erwärmt, und die restliche Lösung des Säurechlorids wurde kontinuierlich innerhalb von 12 Stunden zugetropft. Anschließend wurde wei tere 12 Stunden lang bei 50° C gerührt, dann auf 0° C abgekühlt. Es wurden 50 ml auf 0° C abgekühlte 1N Salzsäure zugesetzt, und es wurde 24 Stunden lang bei Raumtemperatur gerührt. Die wäßrige Phase wurde abgetrennt und zweimal mit jeweils 20 ml Methylenchlorid extrahiert. Die vereinigten organischen Phasen wurden mit 75 ml gesättigter wäßriger Natriumhydrogencarbonat-Lösung, dann mit 50 ml Wasser gewaschen und über Natriumsulfat getrocknet. Nach Entfernen der Lösungsmittel bei 40° C unter vermindertem Druck wurde das Rohprodukt über eine Kieselgelsäule chromatographiert. Es wurden 0,49 g (23 % der Theorie) analysenreines 3,4-Dimethoxyphenyl-(1-methyl-2-phenyl-1-phthalimidoethyl)-keton mit einem Schmelzpunkt von 159-161° C erhalten.

Als Nebenprodukt konnten 0,15 g (10 % der Theorie) analysenreines 2-Methyl-2-phthalimido-1-indanon mit einem Schmelzpunkt von 180° C isoliert werden.

Beispiel 15:

Eine Lösung von 3,25 g (10,0 mMol) N-Phthaloyl-O-methyl-(S)-tyrosin in 50 ml 1,2-Dichlorethan wurde bei 0° C mit 2,29 g (11,0 mMol) Phosphorpentachlorid versetzt und zunächst 30 Minuten lang bei 0° C, dann 16 Stunden lang bei Raumtemperatur gerührt. Nach Zusatz von weiteren 50 ml 1,2-Dichlorethan wurde ein Zehntel der Lösung schnell zu einer gerührten Lösung von 1,38 g (10,0 mMol) Brenzcatechindimethylether und 160 mg Eisen-(III)-chlorid (10 Molprozent) in 70 ml 1,2-Dichlorethan zugegeben. Das Reaktionsgemisch wurde auf 50° C erwärmt, und die restliche Lösung des Säurechlorids wurde kontinuierlich innerhalb von 12 Stunden zugetropft. Anschließend wurde weitere 12 Stunden lang bei 50° C gerührt, dann auf 0° C abgekühlt. Es wurden 100 ml auf 0° C abgekühlte 1N Salzsäure zugesetzt, und es wurde 24 Stunden lang bei Raumtemperatur gerührt. Die wäßrige Phase wurde abgetrennt und zweimal mit jeweils 40 ml Methylenchlorid extrahiert. Die vereinigten organischen Phasen wurden mit 150 ml gesättigter wäßriger Natriumhydrogencarbonat-Lösung, dann mit 100 ml Wasser gewaschen und über Natriumsulfat getrocknet. Nach Entfernen der Lösungsmittel bei 40° C unter vermindertem Druck wurde das Rohprodukt über eine Kieselgelsäule chromatogra phiert. Es wurden 1,93 g (43 % der Theorie) analysenreines (S)-3,4-Dimethoxyphenyl-[2-(4-methoxyphenyl)-1-phthalimidoethyl] -keton als Öl erhalten.

$[\alpha]_D^{20}$ : -245,2° (c = 1, $CHCl_3$).

Als Nebenprodukt konnten 0,42 g (14 % der Theorie) analysenreines 6-Methoxy-2-phthalimido-1-indanon mit einem Schmelzpunkt von 197-199 °C isoliert werden.

## Ansprüche

1. Verfahren zur Herstellung von Aryl-(1-phthalimido)-alkyl ketonen der allgemeinen Formel

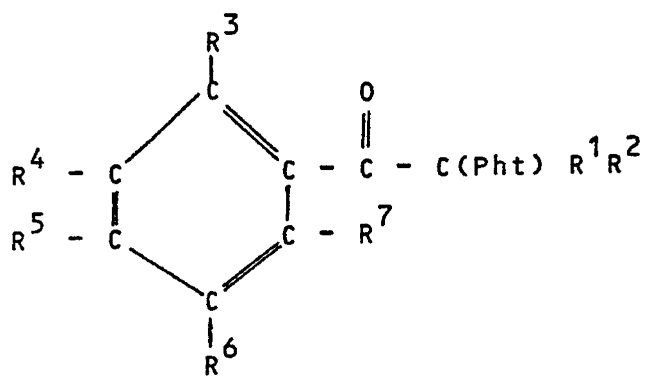

(I),

in der

$R^1$ Wasserstoff oder einen geradkettigen oder verzweigten ($C_1$-$C_4$)-Alkylrest,

$R^2$ Wasserstoff, einen geradkettigen oder verzweigten ($C_1$-$C_6$)-Alkylrest, einen Benzylrest, einen 4-($C_1$-$C_4$)-Alkoxybenzylrest, einen 4-Benzyloxylbenzylrest, einen 3,4-Di-($C_1$-$C_4$)-alkoxybenzylrest, einen 1,3-Benzodioxol-5-yl-methylrest, einen N-Formyl-indol-3-yl-methylrest, einen N-($C_2$-$C_3$)-Acylindol-3-yl-methylrest, einen 5-($C_1$-$C_4$)-Alkoxy-N-($C_2$-$C_3$)-acyl-indol-3-yl-methylrest, einen 5-($C_1$-$C_4$)-Alkoxy-N-formyl-indol-3-yl-methylrest oder einen durch eine ($C_1$-$C_6$)-Alkoxygruppe, eine Benzyloxygruppe, eine Phenyloxygruppe, eine Phthalimidogruppe, eine ($C_1$-$C_4$)- Alkoxycarbonylgruppe oder eine Benzyloxycarbonylgruppe substituierten ($C_1$-$C_4$)-Alkylrest,

$R^3$,$R^4$,$R^5$,$R^6$ und $R^7$ unabhängig voneinander Wasserstoff, einen geradkettigen oder verzweigten ($C_1$-$C_4$)-Alkylrest, einen ($C_1$-$C_6$)-Alkoxyrest, einen Benzyloxyrest oder einen Phenyloxyrest und

Pht einen Phthalimidorest bedeuten,

dadurch gekennzeichnet, daß man eine aromatische Verbindung der allgemeinen Formel

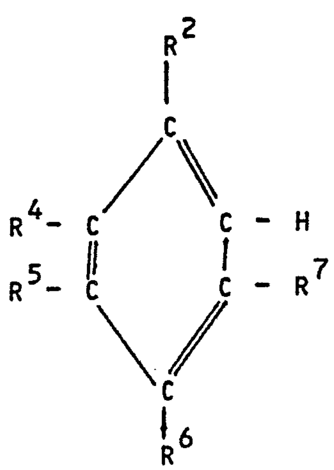

(II),

in der $R^3$, $R^4$, $R^5$, $R^6$ und $R^7$ jeweils eine der bereits genannten Bedeutungen aufweisen, in einem inerten Lösungsmittel und in Gegenwart von Eisen-(III)-chlorid mit einem N-Phthaloyl-2-aminocarbonsäurechlorid der allgemeinen Formel

$$Cl - \overset{O}{\overset{\|}{C}} - C\,(Pht)\,R^1R^2 \qquad (III),$$

in der $R^1$,$R^2$ und Pht die bereits genannten Bedeutungen aufweisen, umsetzt.

2. Verfahren nach Anspruch 1, dadurch gekennzeichnet, daß man das Eisen-(III)-chorid in einer Menge zwischen 0,5 und 50 Molprozent, bezogen auf das eingesetzte N-Phthaloyl-2-aminocarbonsäurechlorid der allgemeinen Formel (III) einsetzt.

3. Verfahren nach Anspruch 1 oder 2, dadurch gekennzeichnet, daß man die Umsetzung bei einer Temperatur im Bereich von -10 bis 80 °C vornimmt.

4. Verfahren nach einem der Ansprüche 1 bis 3, dadurch gekennzeichnet, daß man das N-Phthaloyl-2-aminocarbonsäurechlorid der allgemeinen Formel (III) in dem inerten Lösungsmittel aus der entsprechenden N-Phthaloyl-2-aminocarbonsäure herstellt und dann das Eisen-(III)-chlorid und die aromatische Verbindung der allgemeinen Formel (II) hinzugibt.